# EUROPEAN PATENT APPLICATION

(11) **EP 0 845 454 A1**
(43) Date of publication of application: **03.06.1998**
(21) Application number: 97309596.1
(22) Date of filing: 27.11.1997
(51) Int. Cl.: C07C 69/757, C07C 211/27, C07D 307/93

(54) **Method for the production of optically active cyclopropane derivatives**

(30) Priority: 02.12.1996 JP 321387/96
(71) Applicant: Ajinomoto Co., Inc., Tokyo 104 (JP)
(72) Inventor: Sakata, Katsutoshi, c/o Ajinomoto Co., Inc., Kawasaki-shi, Kanagawa-ken (JP); Tsuji, Takashi, c/o Ajinomoto Co., Inc., Kawasaki-shi, Kanagawa-ken (JP); Kataoka, Noriyasu, c/o Ajinomoto Co., Inc., Kawasaki-shi, Kanagawa-ken (JP); Yatagai, Masanobu, c/o Ajinomoto Co., Inc., Kawasaki-shi, Kanagawa-ken (JP)
(74) Representative: Nicholls, Kathryn Margaret

(57) **Abstract**

A single enantiomer of N-benzyl-α-phenylethylamine is used as a resolving agent for racemic 1-alkoxycarbonyl-2-hydroxymethylcyclopropane-1-carboxylic acid. The latter may be produced by adding an equivalent amount of an alkali to 3-oxa-2-oxobicyclo[3.1.0]cyclopropane-1-carboxylic acid ester in solution. Reaction of the amine and carboxylic acid takes place in a selected solvent, and the resulting diastereomer salt which crystallises but may be filtered off. This may be subsequently decomposed with an alkali, and the resolution agent extracted into an organic solvent and then recovered. Thereafter, the aqueous phase is adjusted to an acid pH, to recover 3-oxa-2-oxobicyclo[3.1.0]hexane-1-carboxylic acid ester at a high optical purity, through solvent extraction.

## Description

The present invention relates to a method for producing optically active cyclopropane derivatives, in particular 1-alkoxycarbonyl-2-hydroxymethyl-cyclopropane-1-carboxylic acid, which is an intermediate useful for producing compounds such as amino acids of non-natural types and anti-viral agents.

It has been known that optically active 3-oxa-2-oxobicyclo[3.1.0]hexane-1-carboxylic acid ester represented by the following general formula (I) is a raw material useful for producing amino acids of non-natural types and anti-viral agents (Helvetica Chimica Acta Vol. 72, 1301 - 1310 (1989); Japanese patent Laid-open No. 5 - 78357). 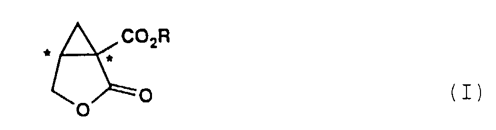 (wherein R represents an alkyl group with 1 to 10 carbon atoms, which may or may not be substituted with an aryl group; and * represents asymmetric carbon).

The compound can be produced readily, by reacting optically active epichlorohydrin with malonate diester. Additionally, a production method from optically active glycidol triflate and malonate diester has been known (J. Org. Chem., Vol. 58, 3767 - 3768 (1993)). However, optically active epichlorohydrin and glycidol triflate to be used according to these methods are so extremely expensive that they are not suitable for industrial production.

A method for producing an optical active substance through the action of esterase on its racemic substance which can be produced inexpensively, to thereby selectively hydrolyze the racemic substance, has been reported (Synthetic Commun., Vol. 21, 1429 - 1432 (1991)), but the procedures therefor are so complex that the method cannot be considered as a satisfactory method.

In one aspect, the present invention provides a production method for optically active 3-oxa-2-oxobicyclo[3.1.0] hexane-1-carboxylic acid ester or an optically active 1-alkoxycarbonyl-2-hydroxymethyl-cyclopropane-1-carboxylic acid as the equivalent substance thereof.

The inventors have found that optically active 3-oxa-2-oxobicyclo [3.1.0] hexane-1-carboxylic acid ester (I) can be produced, by adding an equal amount of a base to racemic 3-oxa-2-bicyclo[3.1.0] hexane-1-carboxylic acid ester represented by the following general formula (II); 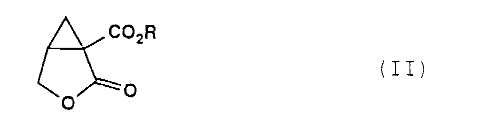
(wherein R represents an alkyl group with 1 to 10 carbon atoms, which may or may not be substituted with an aryl group),
thereby selectively hydrolyzing only the lactone part thereof to produce 1-alkoxycarbonyl-2-hydroxymethylcyclopropane-1-carboxylic acid salt represented by the following general formula (III); 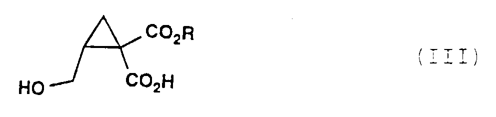
(wherein R represents an alkyl group with 1 to 10 carbon atoms, which may or may not be substituted with an aryl group),
additionally reacting one of optically active forms of N-benzyl-α-phenylethylamine with the resulting product, to crystallize only one of the enantiomers as 1-alkoxycarbonyl-2-hydroxymethylcyclopropane-1-carboxylic acid • N-benzyl-α-phenylethylamine diastereomer salt,
and releasing optically active 1-alkoxycarbonyl-2-hydroxymethylcyclopropane-1-carboxylic acid salt from the crystallized salt by using a base in an aqueous solution, to thereafter remove N-benzyl-α-phenylethylamine, acidifying the resulting solution to thereby form lactone again to generate optically active 3-oxa-2-oxobicyclo[3.1.0]hexane-1-carboxylic acid ester(I). Based on this finding, the present invention has been achieved.

In other words, the present invention is a production method of 1-alkoxycarbonyl-2-hydroxymethylcyclopropane-1-carboxylic acid•N-benzyl-α-phenylethylamine diastereomer salt represented by the following general formula (IV), through the reaction of 1-alkoxycarbonyl-2-hydroxymethylcyclopropane-1-carboxylic acid represented by the following general formula (III) or a salt thereof with optically active N-benzyl-α-phenylethylamine or a salt thereof in a solvent; 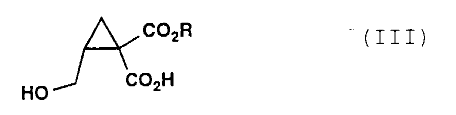
(wherein R represents an alkyl group with 1 to 10 carbon atoms, which may or may not be substituted with an aryl group)
to deposit crystal and isolate the crystal; 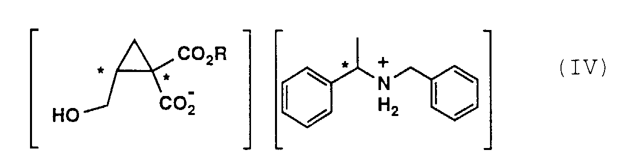
(wherein R represents the same meaning as described above; and * represents asymmetric atom).

Embodiments of the invention are described below by way of example only.

Racemic 1-alkoxycarbonyl-2-hydroxymethylcyclopropane-1-carboxylic acid (III) as a raw material in accordance with the present invention may be produced by adding racemic 3-oxa-2-oxobicyclo[3.1.0]hexane-1-carboxylic acid ester represented by the following general formula (II) to a solvent and adding a base to the resulting solution to hydrolyze only the lactone part. 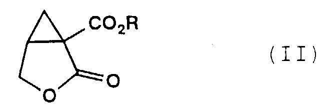 (wherein R represents the same meaning as described above).

In this case, the base may be added in advance to the solvent, satisfactorily. The solvent preferably includes water or a lower alcohol soluble in water or a mixture solvent of the two. Further, the base includes alkali hydroxide, metal alcoholate, tertiary or quaternary amine, specifically including sodium hydroxide, potassium hydroxide, sodium methoxide, potassium methoxide, sodium ethoxide, potassium ethoxide, potassium-t-butoxide, triethylamine, diisopropylethylamine, tetramethylammonium hydroxide and the like.

As to the amount of the base to be used, then, the base is satisfactorily added at 0.9 to 1.1 equivalents to 1 equivalent of racemic 3-oxa-2-oxobicyclo[3.1.0]hexane-1-carboxylic acid ester. If the amount is too much, the ester is hydrolyzed; while if the amount is less, the hydrolysis of the lactone insufficiently progresses, with the resultant reduction of the yield. Practically, the reaction with 1 equivalent is satisfactory.

The hydrolysis is completed under agitation at a temperature of - 10 °C to 60 °C for 5 minutes to 48 hours.

Herein, the racemic 3-oxa-2-oxobicyclo[3.1.0]hexane-1-carboxylic acid ester (II) may be produced through the reaction of epichlorohydrin with malonate diester in the presence of bases such as sodium alkoxide according to the method described in a reference (Helvetica Chimica Acta Vol. 72, 1301 - 1310 (1989)). The ester includes alkyl esters such as methyl, ethyl and butyl, which may or may not be substituted with an aryl group such as phenyl group and tosyl group.

It might be expected that 3-oxa-2-oxobicyclo[3.1.0]hexane-1-carboxylic acid ester has four optically active forms, but only two forms are present, wherein the lactone part is present in the cis configuration to the cyclohexane ring. Because of steric hindrance, compounds with a lactone ring present in the trans configuration cannot exist (Helvetica Chimica Acta Vol. 72, 1301 - 1310 (1989)). Therefore, 1-alkoxycarbonyl-2-hydroxymethylcyclopropane-1-carboxylic acid produced by the hydrolysis of the lactone ring has only two optical isomers ((1R, 2R) -1-alkoxycarbonyl-2-hydroxymethylcyclopropane-1-carboxylic acid and (1S, 2S)-1-alkoxycarbonyl-2-hydroxymethylcyclopropane-1-carboxylic acid).

When produced by the method described above, it is believed that the racemic 1-alkoxycarbonyl-2-hydroxymethylcyclopropane-1-carboxylic acid (III) as a raw material in accordance with the present invention is generally present as the salt of a base (alkali metal, alkali earth metal, amine salt, etc.) used for the hydrolysis, in a reaction solvent, and the salt may satisfactorily be used as starting material for the next step.
Practically, the reaction solution thus recovered is generally used as it is.

1-Alkoxycarbonyl-2-hydroxymethylcyclopropane-1-carboxylic acid•N-benzyl-α-phenylethylamine diastereomer salt (IV) may be isolated by reacting the racemic 1-alkoxycarbonyl-2-hydroxymethylcyclopropane-1-carboxylic acid with optically active N-benzyl-α-phenylethylamine in the solvent described above and filtering the resulting deposited crystal.

When N-benzyl-α-phenylethylamine is used as the free form, the crystal may be recovered by neutralizing the base used for the hydrolysis of the lactone ring with acids such as hydrochloric acid and sulfuric acid, but if the salt of N-benzyl-α-phenylethylamine is used, such acid neutralization is not necessary.

From the respect of procedures and yield, the concentration of the racemic 1-alkoxycarbonyl-2-hydroxymethylcyclopropane-1-carboxylic acid is preferably 0.1 to 3.0 M. The reaction is carried out at room temperature or under heating, and the resulting deposited crystal is cooled and thereafter filtered. By preliminarily adding the crystal of the objective 1-alkoxycarbonyl-2-hydroxymethylcyclopropane-1-carboxylic acid•N-benzyl-α-phenylethylamine diastereomer salt into the reaction solution, then, crystallization thereof can be promoted.

The amount of N-benzyl-α-phenylethylamine or a salt thereof to be used is not specifically limited, but 0.4 to 1.5 equivalents, preferably 0.5 to 1.2 equivalents thereof is used per the racemic 1-alkoxycarbonyl-2-hydroxymethylcyclopropane-1-carboxylic acid (or 3-oxa-2-oxobicyclo[3.1.0]hexane-1-carboxylic acid ester).

For the optically active N-benzyl-α-phenylethylamine to be used as a resolution agent, in accordance with the present invention, (R)-(+) form or (S)-(-) form is present, and any of the forms can be produced by a method described in Journal fur Praktische Chemie [2], 86, 287 (1912). These may be used in the form of salts such as hydrochloride and sulfate.

When the (R)-(+) form is used, (1R, 2R)-1-alkoxycarbonyl-2-hydroxymethylcyclopropane-1-carboxylic acid• (R) - (+) -N- benzyl-α-phenylethylamine diastereomer salt (IVa) is recovered as crystal; when the (S)-(-) form is used, (1S, 2S)-1-alkoxycarbonyl-2-hydroxymethylcyclopropane-1-carboxylic acid• (S) - (-) -N-benzyl-α-phenylethylamine diastereomer salt (IVb) is recovered as crystal. 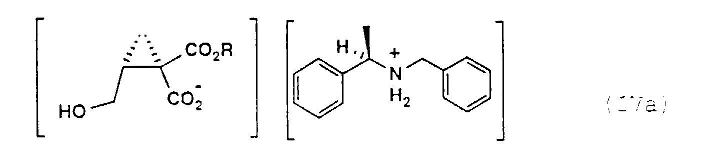 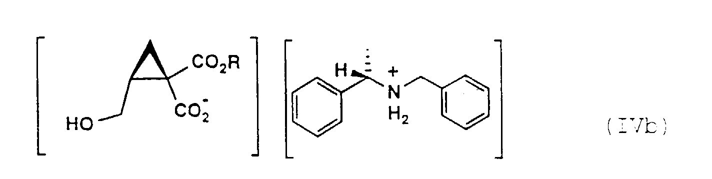 (wherein R represents an alkyl group with 1 to 10 carbon atoms, which may or may not be substituted with an aryl group).

The recovered crystal may be used satisfactorily as raw material for an objective compound, but the crystal can be converted into optically active 3-oxa-2 oxobicyclo[3.1.0]hexane-1-carboxylic acid ester (I), by decomposing the diastereomer salt by using an alkali such as sodium hydroxide, lithium hydroxide, and potassium hydroxide, extracting and recovering N-benzyl-α-phenylethylamine in an organic solvent such as chloroform, ethyl acetate and diethyl ether, making the aqueous phase acidic by using hydrochloric acid, sulfuric acid, methanesulfonic acid, benzenesulfonic acid, toluenesulfonic acid and the like, to close the lactone ring, prior to appropriate extraction in a solvent.

The optically active 3-oxa-2-oxobicyclo[3.1.0]hexane-1-caboxylic acid ester thus recovered displays good optical purity, and the ester at a higher purity can be recovered, if necessary, by repeating the procedures described above or by re-crystallizing 1-alkoxycarbonyl-2-hydroxymethylcyclopropane-1-carboxylic acid•N-benzyl-α-phenylethylamine diastereomer salt (IV).

Furthermore, the filtrate recovered after 1-alkoxycarbonyl-2-hydroxymethylcyclopropane-1-carboxylic acid•N-benzyl-α-phenylethylamine diastereomer salt (IV) is filtered off contains an enantiomer reverse to the crystallized substance, and therefore, the reverse enantiomer can be recovered from the filtrate as well. More specifically, by adjusting the filtrate to acidity, 3-oxa-2-oxobicyclo[3.1.0]hexane-1-carboxylic acid ester is extracted in an appropriate organic solvent. In this case, by once adjusting the filtrate to alkalinity to preliminarily extract the amine as the resolution agent, then, the resulting solution is again adjusted to acidity, to extract and recover 3-oxa-2-oxobicyclo[3.1.0]hexane-1-carboxylic acid ester.

The substrate compound represented by the general formula (III) described above to be optically resolved by using N- benzyl-α-phenylethylamine as a resolution agent by the method of the present invention is not necessarily in the form of an equimolar mixture of two optical enantiomers (complete racemic mixture).

The substrate compound represented by the general formula (III) to be subjected to optical resolution by using N- benzyl-α-phenylethylamine as a resolution agent by the method of the present invention is not necessarily pure.

### [Examples]

The present invention will now be described in detail in examples, but the scope of the present invention is not limited to the examples.

### [Example 1]

Racemic ethyl 3-oxa-2-oxobicyclo[3.1.0]hexane-1-carboxylate (1.70 g; 10 mmol) was added to an aqueous 1N sodium hydroxide solution (10 ml) under cooling in ice and water, followed by agitation at room temperature for one hour. Under agitation at room temperature, (R)-(+)-N-benzyl-α-phenylethylamine (2.1 g (10 mmol); Yamakawa Pharmaceutical Product Industry) was added to the resulting mixture, followed by addition of 1 N hydrochloric acid (10 ml), for agitation in a water bath at 10 °C for 4 hours. The crystal was filtered and dried in vacuum, to recover (1R, 2R)-1-ethoxycarbonyl-2-hydroxymethylcyclopropane-1-carboxylic acid• (R) - (+) -N- benzyl-α-phenylethylamine salt (1.75 g).

A part of the crystal was dissolved in chloroform, followed by extraction of the component amine in 2N hydrochloric acid, to modify 1-ethoxycarbonyl-2-hydroxymethylcyclopropane-1-carboxylic acid to ethyl 3-oxa-2-oxobicyclo [3.1.0]hexane-1-carboxylate, to determine the purity of the carboxylate ethyl in the form of (1S, 2R), which was 89.2 % ee. The optical purity thereof was measured by gas chromatography, using CP-Chirasil-Dex CB (25 m x 0.25 mm; I.D. 0.25 µm) at 140 °C, while monitoring with a hydrogen flame ionization detector.

The remaining (1R, 2R)-1-ethoxycarbonyl-2-hydroxymethylcyclopropane-1-carboxylic acid• (R) - (+)-N-benzy 1-α-phenylethylamine salt (1.75 g) was dissolved in chloroform (25 ml), followed by extraction in an aqueous 0.5 N sodium hydroxide solution (9.2 ml). After adding 2N hydrochloric acid to the aqueous phase to adjust the phase to pH 1, chloroform extraction was twice repeated. The organic phase was dried over anhydrous magnesium sulfate, followed by concentration and drying under reduced pressure, to recover ethyl(1S, 2R)-3-oxa-2-oxobicyclo[3.1.0]hexane-1-carboxylate (672 mg). This corresponds to a yield of 39.5 %. The optical purity thereof was 91 % ee.

An aqueous 0.5N sodium hydroxide solution (10.8 ml) was added to the resulting filtrate, followed by chloroform extraction twice. By adding 2N hydrochloric acid (14 ml) to the aqueous phase to adjust the phase to pH 1, chloroform extraction was repeated twice. The organic phase was dried over anhydrous magnesium sulfate, followed by concentration and drying under reduced pressure, to recover the filtered ethyl (1R, 2S)-3-oxa-2-oxobicyclo[3.1.0]hexane-1-carboxylat e(842 mg). This corresponds to a yield of 49.5 %. Additionally, the optical purity thereof was 77 % ee.

The remaining chloroform extracts (left after the sodium hydroxide solution extractions) were combined together, and the resulting solution was dried over anhydrous magnesium sulfate, followed by concentration and drying under reduced pressure, to recover crude (R)-(+)-N-benzyl-α-phenylethylam ine (2.27 g). The phenylethylamine was dissolved in ethyl ether (10 ml), followed by addition of a solution of 4N hydrochloric acid in dioxane (2.65 ml) under agitation and cooling in ice and water for 2 hours. The crystal was filtered and dried in vacuum, to recover (R)-(+)-N-benzyl-α -phenylethylamine hydrochloride salt (2.21 g) . This corresponds to a yield of 93.7 %.

### [Example 2]

A crude solution of racemic ethyl 3-oxa-2-oxobicyclo[3.1.0]hexane-1-carboxylate at a content of 43.6 % (5.01 g; 2.18 g of lactone, 12.8 mmol) was added to an aqueous 0.6N sodium hydroxide solution (25 ml) under agitation and cooling in ice and water, followed by agitation at room temperature for one hour. After extraction twice in chloroform, (R)-(+)-N-benzyl-α-phenylethylamine hydrochloride salt (1.70 g) was added in portions to the aqueous phase under agitation at room temperature. After agitation at room temperature for 30 minutes, the resulting mixture was agitated in a water bath at 50 °C for 30 minutes. Subsequently, the mixture was agitated overnight at 4 °C, to recover the crystal, followed by drying in vacuum, to recover (1R, 2R) -1-ethoxycarbonyl-2-hydroxymethylcyclopropane-1-carboxylic acid• (R) - (+) -N-benzyl-α-phenylethylamine salt (2.00 g). By the same method as in Example 1, the optical purity thereof was determined. The optical purity of the (1R, 2R) form thereof was 90.2 % ee.

The resulting (1R, 2R) -1-ethoxycarbonyl-2-hydroxymethylcyclopropane-1-carboxylic acid• (R)-(+)-N-benzy 1-α-phenylethylamine salt (2.00 g) was dissolved in chloroform (40 ml) and was extracted in an aqueous 0.6N sodium hydroxide solution (9.0 ml). By adding 2N hydrochloric acid (10 ml) to the aqueous phase to adjust the phase to pH 1, chloroform extraction was done twice. The organic phase was dried over anhydrous magnesium sulfate, followed by concentration and drying under reduced pressure, to recover ethyl (1S, 2R)-3-oxa-2-oxobicyclo[3.1.0]hexane-1 -carboxylate(881 mg). This corresponds to a yield of 40.5 %. The optical purity thereof was 90 % ee.

### [Example 3]

Racemic methyl 3-oxa-2-oxobicyclo[3.1.0]hexane-1-carboxylate(1.644 g; 10.53 mmol) was added to an aqueous 1N sodium hydroxide solution (10.53 ml) under cooling in ice and water. While agitation at room temperature, (R)-(+)-N-benzyl-α-phenylethylamine (2.241 g; 10.53 mmol) was added to the resulting mixture, followed by addition of 1 N hydrochloric acid (10.48 ml), and then, the crystal of (1R, 2R)-2-hydroxymethyl-1-methoxycarbonylcyclopropane-1-carboxylic acid•(R)-(+)-N- benzyl-α-phenylethylamine salt was seeded into the resulting mixture and left to stand overnight under cooling in ice and water. The crystal was filtered and dried in vacuum, to recover (1R, 2R)-2-hydroxymethyl-1-ethoxycarbonylcyclopropane-1-carboxylic acid• (R) - (+) -N-benzyl-α-phenylethylamine salt (1.42 g). The optical purity of the (1R, 2R) form thereof was 82 % ee.

### [Example 4]

Racemic methyl 3-oxa-2-oxobicyclo[3.1.0]hexane-1-carboxylate (1.644 g; 10.53 mmol) and 2N sodium hydroxide solution in methanol (6.31 ml) were added to methanol (5.7 ml) under cooling in ice and water. While agitation at room temperature, (R) - (+) -N- benzyl-α-phenylethylamine (2.72 g; 12.80 mmol) was added to the resulting mixture, followed by addition of 6 N hydrochloric acid (2.10 ml), and then, the crystal 1 of (1R, 2R)-2-hydroxymethyl-1-methoxycarbonylcyclopropane-1-carboxylic acid• (R) - (+) -N-benzyl-α-phenylethylamine salt was seeded into the resulting mixture and left to stand overnight under cooling in ice and water. The crystal was filtered and dried in vacuum, to recover (1R, 2R)-2-hydroxymethyl-1-methoxycarbonylcyclopropane-1-carboxylic acid• (R) - (+) -N-benzyl-α-phenylethylamine salt (1.62 g). The optical purity of the (1R, 2R) form thereof was 95 % ee.

### [Example 5]

Racemic methyl 3-oxa-2-oxobicyclo[3.1.0]hexane-1-carboxylate (1.28 g; 8.20 mmol) and 2N sodium hydroxide solution in methanol (4.1 ml) were added to methanol (3.7 ml) under cooling in ice and water. While agitation at room temperature, (R)-(+)-N-benzyl-α-phenylethylamine hydrochloride salt (2.03 g; 8.20 mmol) was added to the resulting mixture, and then, the crystal of (1R, 2R)-2-hydroxymethyl-1-methoxycarbonylcyclopropane-1-carboxylic acid• (R)- (+)-N-benzyl-α-phenylethylamine salt was seeded into the resulting mixture and left to stand overnight under cooling in ice and water. The crystal was filtered and dried in vacuum, to recover (1R, 2R)-2-hydroxymethyl-1-methoxycarbonylcyclopropane-1-carboxylic acid• (R) - (+) -N-benzyl-α-phenylethylamine salt (1.12 g). The optical purity of the (1R, 2R) form thereof was 91 % ee.

## Claims

1. A method for the production of 1-alkoxycarbonyl-2-hydroxymethylcyclopropane-1-carboxylic acid • N-benzyl-α-phenylethylamine diastereomer salt represented by the following general formula (IV), comprising reacting optically active N-benzyl-α-phenylethylamine or a salt thereof with 1-alkoxycarbonyl-2-hydroxymethylcyclopropane-1-carboxylic acid represented by the following general formula (III) or a salt thereof in a solvent thereby depositing crystals and separating the crystals; 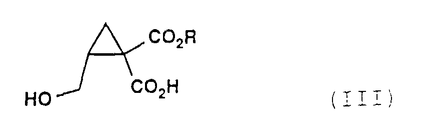 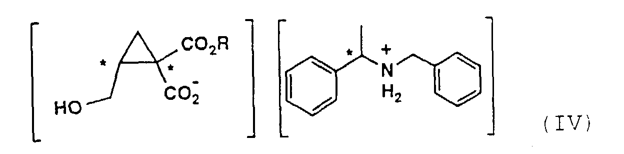 (wherein R represents an alkyl group with 1 to 10 carbon atoms, which may or may not be substituted with an aryl group; and * denotes an asymmetric carbon).

2. A method according to claim 1, wherein the solvent is any of water, a lower alcohol or a mixture of water with a lower alcohol.

3. A production method according to claim 1 or 2, wherein the 1-alkoxycarbonyl-2-hydroxymethylcyclopropane-1-carboxylic acid is 1-ethoxycarbonyl-2-hydroxymethylcyclopropane-1-carboxylic acid or 2-hydroxymethyl-1-methoxycarbonylcyclopropane-1-carboxylic acid.

4. A method according to any one of the preceding claims for the production of (1R, 2R)-1-alkoxycarbonyl-2-hydroxymethylcyclopropane-1-carboxylic acid• (R) - (+) -N-benzyl-α-phenylethylamine salt represented by the following general formula (IVa); 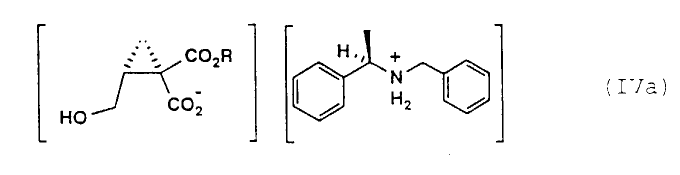 the method comprising reacting 1-alkoxycarbonyl-2-hydroxymethylcyclopropane-1-carboxylic acid or a salt thereof with (R)-(+)-N-benzyl-α-phenylethylamine or a salt thereof.

5. A method according to any one of claims 1 to 3 for the production of (1S, 2S ) -1-alkoxycarbonyl-2-hydroxymethylcyclopropane-1-carboxylic acid• (S)-(-) -N-benzyl-α-phenylethylamine salt represented by the following general formula (IVb); 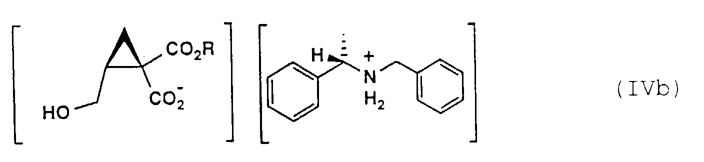 the method comprising reacting 1-alkoxycarbonyl-2-hydroxymethylcyclopropane-1-carboxylic acid or a salt thereof with (S)-(-)-N-benzyl-α-phenylethylamine or a salt thereof.

6. A method for the production of optically active 3-oxa-2-oxobicyclo[3.1.0]hexane-1-carboxylic acid ester represented by the following general formula (1); 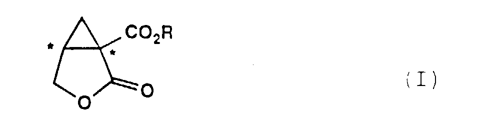
(wherein R represents an alkyl group with 1 to 10 carbon atoms, which may or may not be substituted with an aryl group; and * represents asymmetric carbon),
comprising removing N-benzyl-α-phenylethylamine from 1-alkoxycarbonyl-2-hydroxymethylcyclopropane-1-carboxylic acid•N-benzyl-α-phenylethylamine diastereomer salt recovered by a method according to any one of claims 1 to 5, to recover optically active 1-alkoxycarbonyl-2-hydroxymethylcyclopropane-1-carboxylic acid, and subjecting the carboxyl group and the hydroxy group to a ring closing reaction to prepare a lactone ring.

7. 1-alkoxycarbonyl-2-hydroxymethylcyclopropane-1 carboxylic acid•N-benzyl-α-phenylethylamine diastereomer salt of formula (IV): 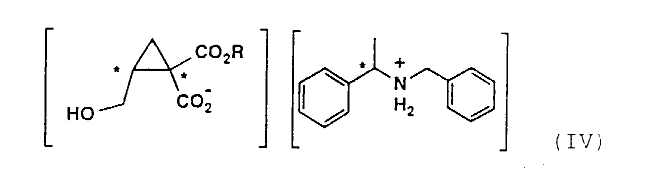 (wherein R represents an alkyl group with 1 to 10 carbon atoms, which may or may not be substituted with an aryl group, and * denotes an asymmetric carbon)

8. (1R, 2R)-1-alkoxycarbonyl-2-hydroxymethylcyclopropane-1-carboxylic acid• (R) - (+) -N-benzyl-α-phenylethylamine salt of formula (IVa.): 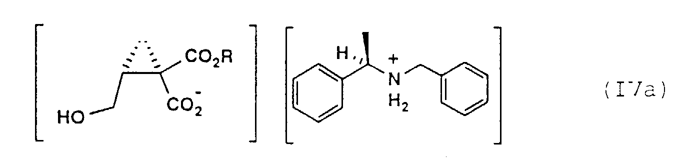 or (1S, 2S)-1-alkoxycarbonyl-2-hydroxymethylcyclopropane-1-carboxylic acid• (S)-(-) -N-benzyl-α-phenylethylamine salt of formula (IVb): 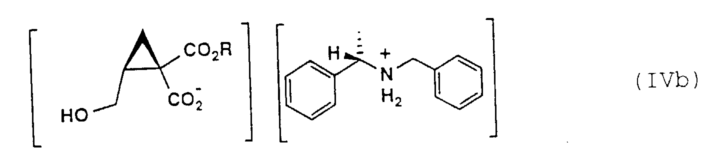 wherein R represents an alkyl group with 1 to 10 carbon atoms, which may or may not be substituted with an aryl group.
